# EUROPÄISCHE PATENTANMELDUNG

(11) **EP 0 884 330 A1**
(43) Veröffentlichungstag der Anmeldung: **16.12.1998**
(21) Anmeldenummer: 97810369.5
(22) Anmeldetag: 12.06.1997
(51) Int. Cl.: C08B 37/00, A23L 1/0526, A61K 31/715, A61K 47/36, A23K 1/16, C09D 105/00, C06B 23/00

(54) **Verfahren zur Herstellung von reinem Guarkernmehl**

(71) Anmelder: Meyhall AG, 8280 Kreuzlingen (CH)
(72) Erfinder: Wielinga, Willem Cor, 8274 Tägerwilen (CH)
(74) Vertreter: Groner, Manfred

(57) **Zusammenfassung**

Gegenstand der vorliegenden Erfindung ist ein Verfahren zur Herstellung von reinem Guarkernmehl, das, wenn es in gelöster Form vorliegt, eine transparente Lösung unterschiedlicher, d.h. geringer bis sehr hoher Viskosität ergibt. Das Verfahren umfasst das Behandeln von Guarsplits mit einer alkalischen Lösung in Anwesenheit von Wasserstoffperoxid, teilweise Neutralisation der Splits mit einer Säure, mechanisches Entfernen der peripheren Zellschichten, Behandeln der Splits mit einer wässrigen Alkohollösung und anschliessendem Vermahlen der gereinigten Splits zu Mehl.

## Beschreibung

Gegenstand der vorliegenden Erfindung ist ein Verfahren zur Herstellung von Guarkernmehl, das, wenn es in Wasser gelöst vorliegt, eine transparente Lösung unterschiedlicher, d.h. geringer bis sehr hoher Viskosität ergibt, wobei das Verfahren trotz extensiver Reinigung gute Ausbeuten des reinen Mehls liefert. Transparente, hochviskose Lösungen aus reinem Guarkernmehl sind vor allem in der Nahrungsmittelindustrie von grosser Bedeutung.

Guarkernmehl findet als Verdickungsmittel im Textil- und Sprengstoffsektor, als Bindemittel in der Papierindustrie, als Flockungsmittel bei der Erzgewinnung und Hilfsmittel bei der Erdgas- und Erdölförderung, im pharmazeutischen und kosmetischen Bereich, und als Verdickungsmittel, Emulgator und Co-Stabilisator im Nahrungsmittelbereich und der Nahrungsmitteltechnologie Verwendung.

In der Pharmazie wird niedrigviskoses Guarkernmehl zur Sprüheinbettung zum Beispiel von Vitaminen verwendet, um deren Lagerstabilität zu erhöhen. Darüberhinaus garantiert die Verwendung von Guarkernmehl in Sprays eine nahezu monomolekulare Verteilung der Wirkstoffe und eine dadurch verbesserte, gleichmässige Resorption, was im Fall von Asthmamitteln und diversen Antiallergika wünschenswert ist. Durch den ausserordentlich geringen Proteingehalt des reinen Guarkernmehls besteht keine Gefahr für die Ausbildung allergischer Reaktion auf ein diese Substanz enthaltendes Medikament. Weitere Anwendungen auf diesem Gebiet sind die Formulierung von Retardtabletten und als Mittel zur Senkung des Cholesterinspiegels. Im medizinischen Bereich wird stark visköses Guarkernmehl auch als Stabilisator in Kontrastmitteln verwendet.

Guarkernmehl hat sich unter anderem auch als ideales diätetisches Mittel erwiesen, da seine Bausteine, die sogenannten Galaktomannane, von den menschlichen Magen- und Dünndarmenzymen nicht angegriffen werden. Dies ist insofern zu erwarten, da im resorbierenden Teil des menschlichen Verdauungssystem weder β-Mannanasen noch β-Mannosidasen oder α-Galaktosidasen vorhanden sind, die zur Aufspaltung dieser Bausteine notwendig wären. Da die Bausteine des Guarkernmehls nicht in den menschlichen Stoffwechsel eingehen, ist das Guarkernmehl in keiner Weise als Kalorienträger oder - lieferant anzusehen. Da sich das Guarkernmehl aus völlig neutralen Polysacchariden, d.h. genauer aus Galaktomannanen, zusammensetzt, die weder Uronsäure noch andere ionogene Gruppen aufweisen, stellen sie in physiologischer Hinsicht völlig unbedenkliches Material dar.

Ein weiterer Vorteil im Hinblick auf seine Verwendung als Nahrungsmittelzusatzstoff ist seine völlige Geschmacksneutralität. Es findet Verwendung in kalorien- oder fettreduzierten Nahrungsmitteln oder Getränken, die von dem Konsumenten häufig als "dünn" empfunden werden. Die Zugabe von reinem Guarkernmehl zu diesen Produkten verleiht ihnen eine "sahnigere" Konsistenz. Bei der Herstellung von Fruchtsäften wird Guarkernmehl verwendet, um die Fruchtpulpe gleichmässig zu resuspendieren, in Puddings und Cremes dient es als Verdickungsmittel, in Eiscremes, Milchshakes, Mousse und ähnlichen Produkten als Stabilisator.

Mit herkömmlichen Guarkernmehlpräparaten war nur eine geringe molekulare Wechselwirkung mit dem Biopolymer Xanthan zu verzeichnen. Beim Mischen dieser beiden Kolloide trat zwar eine synergistische Viskositätserhöhung auf, eine spezifische Gelbildung wie im Fall von Carubin, dem Johannisbrotkernmehl und Xanthan trat jedoch nicht auf. Wenn man Mischungen in einem Verhältnis von 1:1 aus dem Guarkernmehl erhalten gemäss der Erfindung und Xanthan gemeinsam erhitzt und bei 4° C (Kühlschranktemperaturen) abkühlen lässt, bildet sich ein weiches Gel. Ein Vorteil dieser Kombination aus Guarkernmehl und Xanthan besteht darin, dass das Gel aus diesen beiden Komponenten bei Körpertemperatur schmilzt und sich daher hervorragend zur Herstellung von geleeartigen Lebensmitteln, als Trägersubstanz bei der Verabreichung von Medikamenten in Zäpfchenform und ähnlichem eignet. Guarkernmehl und Xanthan werden darüber hinaus gemeinsam als Costabilisatoren bei der Herstellung von Salatdressings verwendet, da diese Kombination, im Gegensatz zu Guarkernmehl, wenn es allein verwendet wird, säureresistenter ist.

Guarkernmehl wird aus dem Endosperm der Guarbohne (*Cyamopsis tetragonobolus*) gewonnen. Guarkernmehl besteht weitgehend aus Galactomannanen, d.h. aus Polysacchariden, deren Hauptkette in 1->4 Richtung durch β-glykosidische Bindungen verknüpft ist, und setzt sich aus Mannose zusammen, die teilweise über primäre OH-Gruppen mit Galaktose verbunden ist. Das Verhältnis von unsubstituierter zu mit Galaktose substituierter Mannose beträgt ca. 2:1, wobei die substituierten Einheiten nicht streng alternierend, sondern in Zweier- oder Dreiergruppen in den Polygalactomannanmolekülen angeordnet sind. Die Guar-Galactomannane bilden schon in geringen Konzentrationen mit Wasser hochviskose Lösungen. 1 gewichtsprozentige Lösungen von handelsüblichem Guarkernmehl in Wasser ergeben Viskositäten von ca. 3'000 bis 6'000 mPa.s.

Guar-Galaktomannane sind aufgrund chemischer und physicochemischer Unterschiede in kaltwasserlösliche, heisswasserlösliche und unlösliche Galaktomannane aufgeteilt worden.

Zur Gewinnung und Reinigung des Guarkernmehls wird die Guar-Saat mechanisch behandelt, wobei ungefähr 35 Teile unreiner Guar-Endospermhälften und ungefähr 60 Teile Guarmehl erhalten werden. Das Guarmehl besteht im wesentlichen aus dem Keim der Saat, der abgeschürften Bohnenschale und kleinen Endospermteilen. Das Endosperm umhüllt den Keim vollständig und wird seinerseits von der Samenschale umschlossen. An den Kontaktstellen zwischen Endosperm und Samenschale befindet sich eine proteinreiche, aleuronähnliche Zellschicht, deren Zellen eng mit dem Endosperm verzahnt sind.

Die unreinen Endospermhälften können weiter mechanisch gereinigt werden und liefern Splits von unterschiedlicher Qualität hinsichtlich ihres Proteingehalts, ihrer durch Säure nicht hydrolisierbaren Bestandteile (A.I.R.) sowie des Schalengehalts. Die in Fachkreisen übliche Bezeichnung "Split" ist dem Begriff "Endospermhälften" gleichzusetzen.

Obwohl Guarkernmehl als Verdickungsmittel bereits eine breite Anwendung findet, ist es erwünscht, seinen Reinheitsgrad und damit verbunden seine physikalischen und physiologischen Eigenschaften zu verbessern. Insbesondere für seine Anwendung im Nahrungsmittelbereich ist die Reinheit des Guarkernmehls von grosser Wichtigkeit. Wünschenswert ist ebenfalls eine bessere Ausnutzung der neutralen, nicht-ionogenen Hauptbestandteile des Endosperms, so dass diese vermehrt anstelle von in Wasser klar löslichen Cellulosederivaten oder anderen Polysacchariden oder in Wasser klar löslichen synthetischen Polymeren in den entsprechenden Industriezweigen angewendet werden können.

Werden die zu Mehl verarbeiteten, zur Zeit auf dem Markt erhältlichen, aus reinem Guarkernmehl bestehenden Produkte in Wasser bei 25° C oder 86 bis 89° C 10 Minuten gelöst, werden trübe Lösungen erhalten. Wird das unlösliche Material dieser Lösungen mit hohen Zentrifugalkräften (>35.000 x g) ausgeschleudert, stellt sich heraus, dass 23 - 35 % des Guarkernmehls aus ausgeschleudertem Material besteht.

Mikroskopische Untersuchungen haben gezeigt, dass sich das ausgeschleuderte Material hauptsächlich aus Schalenfragmenten, Proteinkörpern, unlöslichen peripheren Zellen, intakten, nicht aufgeschlossenen Zellen des inneren Endosperms und anderen Samen- beziehungsweise Splitverunreinigungen zusammensetzt. Eine bekannte chemische Derivatisierung des Guarkernmehls durch Verätherung, Karboxymethylierung, Hydroxypropylierung, deren Kombination sowie Kationisierung ermöglicht die Herstellung von Produkten mit signifikant verbessertem Lösungsverhalten in Wasser und damit einhergehend höherer Transparenz der Lösungen.

Eines der bisher angewendeten Verfahren zur Gewinnung von reinem Guarkernmehl verwendet chlorierte Lösungsmittel, wie zum Beispiel Trichlorethylen (siehe EP 0 130 946, Meyhall Chemical AG). Die Suspension wurde durch einfaches Stehenlassen oder Zentrifugieren fraktioniert, wobei sich eine proteinreiche Fraktion (flottierende Fraktion) ausbildete und sich eine proteinarme Fraktion (Sinkfraktion) abschied.

Es konnte gezeigt werden, dass die obere flottierende Fraktion aus zu Mehl verarbeitetem Endosperm wie Guar CSA 200/50 bis zu 25 % Proteine enthalten kann und die Sinkfraktion, die 75 % des reinen Mehls ausmacht, etwa 1,5 bis 1,6 % Protein enthält. Die Sinkfraktion ist zum Beispiel zur Herstellung von kationischen Derivaten geeignet, die nach ihrer Lösung klare wässrige Lösungen ergeben. Nachteil dieses Verfahrens ist, dass feingemahlene Schalenfragmente ebenfalls in der Sinkfraktion vorgefunden werden. Ein weiterer Nachteil ist die Verwendung von halogenierten Lösungsmitteln, da ein spezifisches Gewicht von 1,47 bis 1,48 kg/l erforderlich ist. Proteine besitzen eine Dichte von 1,3 kg/l und die Galactomannane eine Dichte von 1,5 bis 1,55 kg/l, je nach Feuchtigkeitsgehalt. Das mit dem beschriebenen Verfahren hergestellte Guarkernmehl ist nur für technische Anwendungen geeignet, im Nahrungsmittelbereich ist dieses Guarkernmehl nicht anwendbar, da Reste des verwendeten halogenierten Lösungsmittels, 10 ppb wurden in mit Äthanol extrahierten Fraktionen nachgewiesen, in dem Endprodukt verbleiben. Halogenierte Lösungsmittel sind in unterschiedlichem Mass toxisch und ätzend und besitzen häufig allergisierende Eigenschaften. Auch aus Umweltgründen sollte von diesem Verfahren abgesehen werden.

Ein weiteres Verfahren zur Herstellung von reinem Guarkernmehl wurde bereits 1969 vorgeschlagen. Es bestand aus einer Alkalibehandlung von vorgequollenen Splits bei erhöhten Temperaturen, wobei 100 Teile Alkali von 100 Teilen SPS absorbiert wurden. Die grosse Menge Alkali, d.h. NaOH, musste ausgewaschen werden. Dies wurde mit kaltem Wasser in einem Verhältnis von einem Teil SPS (Single Purified Splits) zu 80 Teilen H₂O und in einem Entwässerungsschritt mit Isopropanol (IPA) durchgeführt, in dem gleichzeitig die restliche NaOH der gereinigten Splits durch Essigsäure neutralisiert wurde.

Nach dem Mahlen wurde ein reines Guarkernmehl von hoher Qualität in einer Ausbeute von 60-70 %, auf dem Rohmaterial SPS (einfach gereinigte Splits) basierend, gewonnen. 1969 wurde dieses Verfahren von Stein, Hall & Co, Long Island City, New York, bis zur Industriereife weiterentwickelt. Das derzeitige Waschverfahren des carboxymethylierten, hydroxypropylierten oder kationisierten Guarkernmehls (Guarether) oder deren Kombinationen mit Wasser beruht auf diesem Verfahren. Der Zweck dieses Reinigungsverfahrens von Guar-Derivaten ist es, Schalenfragmente und periphere Zellschichten zu entfernen, sowie Nebenprodukte der verschiedenen Verätherungsreaktionen (Hydroxypropylierung, Carboxymethylierung und Kationisierung und/oder ihre Kombinationen) zu entfernen.

Ein anderes bekanntes Verfahren zur Herstellung von reinem Guarkernmehl ist die Behandlung der Guar-Splits mit Säure. Dieses Verfahren liefert ein Produkt von ausgezeichneter Qualität, d.h. das resultierende Material ergibt, in Wasser gelöst, Lösungen von grosser Klarheit bei gleichzeitig hoher Viskosität. Ein Nachteil dieses Verfahrens besteht jedoch in der relativ aufwendigen Verfahrensweise mit mehrmaligen Wasch- und Neutralisationsschritten. Darüber hinaus werden für eine Säurebehandlung spezielle Apparaturen benötigt, die das Verfahren sehr kostenintensiv machen.

Trotz der oben beschriebenen extensiven Reinigungsverfahren ist es bislang nicht auf ökonomische und umweltfreundliche Weise gelungen, reines, nicht-derivatisiertes Guarkernmehl zu erhalten, das eine klare, wässrige Lösung mit hoher Viskosität bei gleichzeitig guten Ausbeuten ergibt.

Die Nachteile der bisherigen Verfahrensweisen zur Reinigung und Gewinnung von reinem Guarkernmehl sind:
1. grosse Verluste wertvoller Endospermteile bei der mechanischen Reinigung und dadurch geringe Ausbeuten an reinem Guarkernmehl in Bezug auf das Ausgangsmaterial;
2. Schalenfragmente, die sich noch immer an den verschiedenen Splitqualitäten befinden und in einem grossen Ausmass die Funktionalität der modifizierten Endprodukte stören;
3. periphere, proteinreiche Zellen der Aleuronschicht, die in Wasser kaum quellen und ebenfalls die Funktionalität des Endprodukts negativ beeinflussen;
4. Vorhandensein anderer Verunreinigungen der Guar-Samen, wie Holzpartikel, die nicht vorhanden sein dürfen;
5. hohe Umweltbelastung

Es war daher dringend erwünscht, ein Verfahren zur Herstellung von reinem Guarkernmehl zu entwickeln, das die oben genannten Nachteile beseitigt und reines Guarkernmehl in guten Ausbeuten liefert, das nach seiner Dispersion in Wasser eine klare, hochviskose Lösung ergibt, die vor allem zum Beispiel in der Nahrungsmittelindustrie, pharmazeutische, Farben- und Streichmittelindustrie, sowie bei der Ölgewinnung Anwendung findet.

Ziel der vorliegenden Erfindung ist es, die obengenannten Anforderungen zu erfüllen, d.h. durch ein neues Herstellungsverfahren gute Ausbeuten an reinem, insbesondere für die Nahrungsmittelindustrie geeignetem Guarkernmehl zu erhalten, das niedrig bis hochviskose, klare wässrige Lösungen ergibt.

Das erfindungsgemässe Verfahren zur Herstellung von reinem Guarkernmehl ist in Patentanspruch 1 definiert und umfasst die folgenden Stufen:
(a) Behandeln der Guar-Splits mit einer Base in Anwesenheit von geringen Mengen Wasserstoffperoxid
(b) teilweise Neutralisation der Alkali-Splits mit einer Säure
(c) mechanisches Entfernen der peripheren Zellen
(d) gegebenenfalls zweimaliges Waschen mit Wasser
(e) Behandeln der Splits mit einer wässrigen Alkohollösung

Eine erste Voraussetzung zur Gewinnung reinen Guarkernmehls ist die Verbesserung des Ausgangsmaterials, der sogenannten Splits. Die mit Schale bedeckten Splits machen bis zu 42,5 Gewichtsprozent der Saat aus. Die überlappenden Schalen-Endosperm-Teile, die 13,5 Gewichtsprozent der Saat betragen, sind im wesentlichen in Wasser unlöslich. Der Keimling der Saat umfasst die restlichen 44 %. Diese Mengenangaben zeigen, dass die theoretische Ausbeute an für die Erfindung nutzbaren Splits ohne Schale und ohne überlappende Teile 32 % beträgt.

Das erfindungsgemäss erhaltene reine Guarkernmehl wird am vorteilhaftesten aus Splits hergestellt, die einen Proteingehalt von 4,2 % und einen A.I.R.-Anteil von 1,8 % haben.

Solche Splits können nach einer alkalischen Behandlung unter Verwendung von 10 bis 40 %iger, vorzugsweise 33 % Natronlauge bei Raumtemperaturen, vorzugsweise jedoch bei erhöhten Temperaturen hergestellt werden.

Der Unterschied dieses neuen gegenüber den bisher bekannten Verfahren, die ebenfalls eine Alkalibehandlung des Ausgangsmaterials einschliessen, besteht in der Zugabe von geringen Konzentrationen Wasserstoffperoxid während der Alkalibehandlung. Dieses chemische "Peeling" liefert Splits, von denen die Hüllzellen mit mechanischen Mitteln sehr leicht und nahezu vollständig entfernt werden können. Durch die Behandlung der Splits mit beispielsweise 8-10 Teilen 33 %iger Natronlauge und 1,1 Teilen 35 %igem Wasserstoffperoxid werden die Peripheren Zellen der Splits attackiert und lassen sich durch mechanischen Abrieb entfernen Die Polysaccharide der unter den peripheren Zellen befindlichen Zellschichten werden nicht oxidiert, da die Konzentration des eingesetzten Wasserstoffperoxids zu gering ist.

Die auf diese Weise gereinigten Splits, die wahlweise mit Wasser gewaschen oder auch ungewaschen weiterverarbeitet werden, können für die Zwecke der Erfindung noch signifikant verbessert werden, indem Phospholipide und andere "nicht polare" Substanzen ausgewaschen werden. Dies hat eine grössere Klarheit des in Wasser gelösten Produkts zur Folge und wird durch Behandlung des gereinigten Produkts mit einer wässrigen Alkohollösung, vorzugsweise wässriges Isopropanol (IPA), bei erhöhten Temperaturen erreicht. Bei nicht mit Wasser gewaschenen Produkten ist ein Zusatz weiterer Lauge vor der Behandlung mit Isopropanol nicht nötig, da das Produkt noch ca. 4-7 % NaOH enthält. Wurde das Produkt gewaschen, ist der Zusatz einer alkalischen Lösung erforderlich. Nach der Isopropanolbehandlung werden die alkalischen Splits mit Wasser gewaschen und bei einem gewünschten Feuchtigkeitsgehalt gemahlen.

Der Grad der Befeuchtung während des Mahlens beeinflusst die Eigenschaften des mehligen Endprodukts signifikant. Je höher der Feuchtigkeitsgehalt in einem technisch ausführbaren Mass ist, desto grösser ist die Menge der löslichen Polysaccharide, d.h. um so höher ist die Ausbeute an aktiven Galactomannanen. Dies kann durch die Vergrösserung des Zellvolumens aufgrund des hohen Masses der Befeuchtung erklärt werden. Während des Mahlens werden die gequollenen Zellen durch eine definierte Öffnung oder Spalt gezwungen, wobei die Zellmembran reissen kann, vorausgesetzt, dass die gequollenen Partikel signifikant grösser sind als die Öffnungen (die Elastizität der Zellen spielt ebenfalls eine wichtige Rolle). Bei der Herstellung von Lösungen in Wasser werden die Galaktomannane aus den auf solche Weise zerstörten Zellen freigesetzt, was bei nicht zerstörten Zellen nicht der Fall ist. In diesen Fällen verbleiben die Galactomannane innerhalb der intakten Zellen und tragen nicht wirksam zu der Viskosität der Lösung bei.

Ein Feuchtigkeitsgehalt von ungefähr 82 %, vorzugsweise 72 bis 75 %, ist beim Mahlen aus praktischen und technischen Gründen annehmbar. Feuchtigkeitsgehalte niedriger als 72 % beim Mahlen beeinträchtigen die Qualität des Guarkernmehls. Ein höherer Gehalt als 82 % verursacht technische Probleme.

Ein grosser Vorteil der vorliegenden Erfindung liegt in der 25 %igen Rückgewinnung der abgeriebenen Periperiezellschichten, d.h. in einer extremen Verringerung der Umweltbelastung.

Ein weiterer Vorteil der vorliegenden Erfindung liegt in der Vereinfachung des Verfahrens. Nur noch wenige Schritte sind notwendig, um ein reines Produkt mit hoher Löslichkeit und Viskosität zu erhalten.

Ein weiterer Vorteil der vorliegenden Erfindung besteht in der Möglichkeit, Produkte für Lösungen mit zum Beispiel so niedrigen Viskositäten wie 35 mPa.s und solche mit bis zu 6000 bis 9.000 mPa.s bei 1 %iger Konzentration in Wasser bei 25° C gemessen herzustellen.

Ein weiterer Vorteil der Erfindung besteht darin, reine Guar-Produkte herzustellen, deren Proteingehalt so gering wie 0,2 bis 0,5 % ist.

Der grösste Vorteil der vorliegende Erfindung besteht jedoch darin, dass die bisher üblichen Reinigungsverfahren wesentlich vereinfacht und abgekürzt werden, wodurch die Herstellung des reinen Guarkernmehls wesentlich kostengünstiger wird.

Darüber hinaus ist das neue Verfahren ausgesprochen umweltschonend, da ca. 25 % der abgesiebenen Peripheriezellschichten zurückgewonnen werden können. Der Abrieb, der bei dem hier beschriebenen Verfahren entsteht, kann ausserdem als Verdickungsmittel im Textildruck verwendet werden. Dies bedeutet eine optimale Ausnutzung des Ausgangsmaterials.

Eine Derivatisierung der Galactomannane des Guarkernmehls ist für dessen Kaltwasserlöslichkeit von Bedeutung. Durch die Derivatisierung (z.B. Karboxymethylierung, Hydroxypropylierung, Kationisierung u. ä.) werden eine oder mehrere nicht-ionische, anionische oder kationische Gruppen hinzugefügt, wodurch die verätherten schwierig zugänglichen Galactomannane bereits bei 25° C in Lösung gehen. Die Derivatisierung findet üblicherweise im Anschluss an die Reinigung statt. Die Verwendung des derivatisierten Guarkernmehls ist allerdings in der Lebensmittelindustrie nicht erlaubt. Derivatisiertes, insbesondere kationenaktives, Guarkernmehl findet jedoch zum Beispiel in kosmetischen Produkten, wie Haarconditioner, Körperlotionen und ähnlichem Verwendung.

Das aus der vorliegenden Erfindung resultierende Material ist besonders vorteilhaft, da es in Wasser gelöst, Lösungen von hoher Klarheit ergibt. Eine 1 %ige Lösung (0,9 % Trockensubstanz) des mit diesem neuen Verfahren hergestellten reinen Guarkernmehls zeigt eine Viskosität von 6.000 bis 9.000 mPa.s bei 25° C. Eine Transparenz der wässrigen Lösung von bis zu 95 % kann erzielt werden.

Die Viskosität wurde in einem Brookfield RVT-Viskosimeter, die Transparenz der Lösungen in einem Photospektrometer bestimmt.

Die Erfindung wird im folgenden anhand von einigen Beispielen erläutert. Als Ausgangsmaterial für die beschriebenen Beispiele wurden Splits der höchsten Qualität verwendet.

Aus diesen Beispielen ist ersichtlich, dass eine Behandlung der Peripherie der Guarsplits mit einer optimierten Konzentration an Natronlauge in Anwesenheit von geringen Mengen Wasserstoffperoxid bereits zu reinen Guarprodukten führt.

Diese reinen Produkte zeigen Viskositäten von 5000 bis 9000 mPa.s bei 1 %iger Konzentration sowie Klarheiten von mehr als 80 % bei 0,5 %iger Konzentration und 1 cm Lichtweg bei 500 nm, wohingegen herkömmliche Guar-Produkte in wässriger Lösung Lichttransmissionen von 45 bis 48 % zeigen.

Diese Lichtdurchlässigkeit kann bereits ohne Isopropanolbehandlung erreicht werden. Mit Isopropanol werden Lichtdurchlässigkeiten von bis zu 98 % erzielt, da mit Isopropanol Phospholipide entfernt werden, die durch Waschen mit Wasser nicht zu entfernen sind.

Nach diesem ersten Reinigungsschritt weisen die alkalische Splits einen Wassergehalt von 12 bis 15 % auf.

Die Splits werden zweimal mit Wasser in einem Verhältnis von 1:7 und 1:6 während 6 beziehungsweise 8 Minuten gewaschen. Dabei nehmen die Splits Wasser bis zu 70 % auf und können entweder nach Zugabe von zusätzlichem Wasser bis zu einem Feuchtigkeitsgehalt von 76 bis 78 % gemahlen oder weiter modifiziert werden. Es ist jedoch möglich, die Splits ohne Waschschritt weiter zu behandeln.

Die gemahlenen Produkte besitzen einen Proteingehalt von 1,0 bis 1,2 % und einen A.I.R.-Anteil von ungefähr 0,8. Die Ausbeute dieser Produkte beträgt, je nach Qualität des Ausgangsmaterials, 75 bis 79 %.

### Beispiel I

370 Guar Splits mit einem Galaktomannangehalt von mindestens 84 % werden z.B. in einem vorgeheizten Sigma-Mischer mit 10 % NaOH (84 ml einer 33 %igen NaOH Lösung) und anschliessend nach einer Minute zusätzlich noch mit 4 ml einer 35 %igen H₂O₂ Lösung, die mit 20 ml Isopropanol verdünnt worden war, behandelt. Die Reaktionstemperatur wird indirekt mittels heissem Wasser von 90° C bis auf 70° C erhöht, und dann 30 Minuten konstant gehalten.

Anschliessend wird ein Teil der anwesenden Lauge durch 21 g 96 %ige H₂SO₄, verdünnt mit 8 g Wasser neutralisiert. Der Mischvorgang wird noch 16 Minuten bei 70°C fortgesetzt. Anschliessend werden schnell 70 g Wasser zugegeben, um die behandelten, störenden Peripheriezellschichten während dem Mischvorgang leichter zu entfernen. Der Mischvorgang wird 30 Minuten bei 70° C durchgeführt.

Das Reaktionsgemisch wird durch ein M20-Sieb gesiebt, wobei
- 95 g -M20: (H₂O: 16,2 %) und
- 360 g +M20: (H₂O: 12,9 %)
erhalten werden.

Die +M20-Fraktion zeigt einen NaOH-Gehalt von 5-6 % und wird zweimal mit kaltem Leitungswasser gewaschen, wobei jeweils 6 Teile Leitungswasser auf 1 Teil der ungewaschenen +M20-Fraktion verwendet werden.

Der Waschvorgang wurde 5 bzw. 6 Minute unter intensivem Rühren durchgeführt.

Die gewaschenen, gequollenen Splits werden durch einfaches Sieben zurückgewonnen.

(Die in der Literatur und Patentliteratur erwähnten Alkalibehandlungen unter Anwendung von 20 - 32 % NaOH verlangen eine Waschwassermenge von mehr als 40 Teilen Leitungswasser pro 1 Teil ungewaschenen Splits).

916 g gequollene Splits mit einem Wassergehalt von 73,3 % wurden nach der Reinigung mit Wasser zurückgewonnen.

Eine Viskosität von 7'000 mPa.s wurde bei 20 UpM und 25° C gemessen, nachdem die durch einen Aufschlag erhaltene homogene Lösung der gequollenen Splits über Nacht abgekühlt war.

Die 1 %ige wässrige Lösung wurde in einem Haushalts-Mixer bei höchster Stufe bis etwa 90° C hergestellt. Die zu lösende Menge gequollener Splits wurde berechnet auf ein Wassergehalt von 10 %.

Einer Verdünnung dieser 1 %igen Lösung von 1:1 ergibt bis 500 nm in einer 1cm Küvette eine Lichttransmission von 81,3 %.

N.B.
Werden 40 % weniger NaOH und 40 % weniger H₂SO₄ (unter sonst gleichen Bedingungen) eingesetzt, wird weniger Abrieb (etwa 12 %) und eine signifikant geringere Licht-Transmission von etwa 75 % erhalten.

### Beispiel II

Diese nach Beispiel I erhaltenen Produkte können in ihrer Qualität (Viskosität, Transparenz) signifikant verbessert werden, indem ihr Protein- und A.I.R.-Gehalt weiter gesenkt wird. Produkte, die nicht mit Wasser gewaschen werden, können mit 25 Gewichtsprozent Isopropanol bei 65 bis 70° C ohne Zugabe von zusätzlicher Lauge während 30-60 Minuten behandelt werden, da die Splits noch ungefähr 5-6 % NaOH enthalten. Werden gewaschene Splits verwendet, ist der Zusatz von 2-5 % Lauge, vorzugsweise Natronlauge, erforderlich.

Im Anschluss an die wässrige Isopropanolbehandlung werden die alkalischen Splits gewaschen, durch Zugabe von weiterem Wasser auf den erforderlichen Feuchtigkeitsgehalt gebracht und gemahlen.

Die Transparenz der wässrigen Lösungen solcher Produkte beträgt 93 bis 95 %. Die Viskosität einer 1 % wässrigen Lösung kann je nach Reaktionsbedingung auf 6000 bis 7000 mPa.s eingestellt werden.

Das auf die vorgängig beschriebene Weise gereinigte, als -M20 bezeichnete Produkt ist ein Guarkernmehl mit einer Viskosität von 1000 bis 1500 mPa.s bei 1 %iger Konzentration, das als Basis für signifikant verbesserte Guar-Produkte oder Derivate dienen kann.

Auch die -M20-Fraktion kann auch für Anwendungen aufgearbeitet werden, die alkalische oxidierte Guar-Produkte oder -Derivate verwenden. Ein Anwendungsgebiet wäre zum Beispiel der Polyesterdruck. Durch die Verwendung der -M20-Fraktion erhält das zu bedruckende Material einen weichen Griff, nachdem die verwendeten Dispersionsfarbstoffe bei extrem hohen Temperaturen fixiert worden sind.

### Beispiel III

8522 g Guar-Splits mit einem Galaktomannan-Gehalt von mindestens 84 % werden z.B. in einem indirekt heizbaren Drais-Mischer mit 2580 g einer 33 %igen NaOH-Lösung, die auf 74° C vorgeheizt worden ist, in Kontakt gebracht.

Nach 3 Minuten werden 230 g einer 14 %igen H₂O₂-Lösung langsam zugesetzt.

Die Temperatur des heterogenen Reaktionsgemisches wird bis auf 70° C erhöht und 30 Minute aufrecht gehalten.

Anschliessend wird das Reaktionsgemisch zur besseren Kontrolle der partiellen Neutralisation mit 668 g einer 69,5 %igen Schwefelsäure bis auf 55° C herunter gekühlt.

Die Reaktion wird 30 Minuten bei 70° C fortgesetzt. Die behandelten Guar-Splits werden anschliessend mit kaltem Leitungswasser 5 Minuten gewaschen, wobei 12 Teile Leitungswasser auf 1 Teil ungewaschener Splits angewandt werden. Bei der Reaktion in erwähntem Mischer entsteht praktisch kein Abrieb, so dass auf einem Siebvorgang vor dem Waschen verzichtet werden kann.

Die gewaschenen Splits haben einen Wassergehalt von 72 % und werden in üblicher Weise in einem heissen Luftstrom in einer Hammermühle feiner als M150 gemahlen.

Das gemahlene Produkt enthält noch etwa 2 % NaOH und zeigt eine Viskosität von 4200 mPa.s bei einer Konzentration von 1 %, basierend auf 10 % Wasser bei pH von 6,8.

Die Klarheit einer 0,5 %igen Lösung, die wie beschrieben, gemessen wurde, beträgt 82,4 %.

### Beispiel IV

25 kg Guar Splits mit gleicher, wie in den Beispielen I und II verwendeter Qualität, werden in einem Sigma-Mischer mit 9 % NaOH in Anwesenheit von 0,67 kg einer 14,1 %igen H₂O₂-Lösung, während einer halben Stunde bei 70° C behandelt. Es wurde eine 33 %ige NaOH-Lösung eingesetzt.

Anschliessend wurde während 15 Minuten bei 70° C und offenem Reaktor die Peripheriezellschichten abgerieben. 6,5 gewichtsprozentige -M20 konnten entfernt werden.

Die +M20 Fraktion wurde, wie beschrieben, mit Leitungswasser gewaschen, wobei ein Produkt mit einer Viskosität von 5700 mPa.s (Konzentration 1 %) und einer Klarheit (0,5 %) von 81,3 % erhalten wurde.

Die noch anhaftenden behandelten Peripheriezellschichten können mechanisch durch eine intensive Reibung der +M20 Fraktion z.B. in einer Haushalts-Kaffeemühle, bei niedrigster Mahlgeschwindigkeit abgerieben werden.

Durch wiederholtes 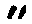 Mahlen 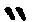 konnten noch zusätzlich etwa 12 % der Peripheriezellschichten entfernt werden, so dass insgesamt etwa 18-19 % der +M20-Fraktion zurückgewonnen wurden.

Diese Periperiezellschichten lassen sich ebenfalls in wässrigen Alkoholen unter intensivem Rühren abreiben.

Wird z.B. 1 Teil des +M20-Fraktion 5 Minuten in 1,2 Teilen 35 gewichtsprozentigem Methylalkohol intensiv gerührt und dieser Vorgang 3 mal wiederholt, so können 13,5 % der +M20-Fraktion zurückgewonnen werden, so dass insgesamt 20 % dieser Fraktion erhalten bleiben.

### Beispiel V

### Kationische Derivatisierung

Das Produkt aus Beispiel III wurde gemahlen und 400 g dieses gemahlenen Produktes wurden in 1600 g eines 25 gewichtsprozentigen wässrigen Isopropanollösung resuspendiert. 100 ml einer 30 gewichtsprozentigen NaOH-Lösung wurden zugegeben. Die Suspension wurde in einer Stickstoffatmosphäre während 30 Minuten unter ständigem Rühren auf 70° C erhitzt. Diese Reaktionstemperatur wurde 1 Stunde aufrechterhalten, dann wurde die Suspension auf 55° C abgekühlt. Das Rühren wurde unterbrochen und nach dem Absetzen des Reaktionsprodukts wurde der Überstand abgegossen.

Das Reaktionsprodukt wurde mit 1000 ml einer 50 Gewichtsprozentigen Isopropanollösung gewaschen und anschliessend mit 10 ml Eisessig behandelt, um eine stöchiometrische Menge NaOH zu neutralisieren.

Der Überstand wurde nach Absetzen des Reaktionsprodukts abgegossen. 7,8 g NaOH wurden auf diese Weise entfernt.

225 g einer 40 gewichtsprozentigen 2,3-Epoxypropyltrimethylammoniumchloriodlösung wurde zugegeben und während 1 Stunde bei 30° C in das alkalisch behandelte Produkt eindringen gelassen. Danach wurden 1100 ml eines 85 gewichtsprozentigen Isopropanollösung als Suspensionsmedium verwendet. Die Stickstoffatmosphäre wurde wieder hergestellt und die Suspension auf 65° C erhitzt. Diese Temperatur wurde 40 Minuten konstant gehalten.

Anschliessend wurde die Stickstoffzufuhr unterbrochen, um das Reaktionsprodukt mit Luftsauerstoff in Kontakt zu bringen und so die endgültige Viskosität des Kationischen Guarproduktes einzustellen.

Die Reaktion wurde 45 Minuten bei 65° c durchgeführt. Das Produkt wurde anschliessend mit 800 ml einer 85 Gewichtsprozentigen Isopropanollösung dann mit 1000 ml dergleichen Lösung gewaschen. Während dem Waschvorgang wurden 25 ml 99 gewichtsprozentiger Eisessig zugegeben, wodurch die Lauge neutralisiert wurde.

Das Produkt wurde durch Filtration zurückgewonnen und mit heisser Luft bei 70° C getrocknet.

Dieses Kationische Guar wies eine Viskosität bei 1 %igen Konzentration in Wasser von 880 mPa.s (auf 10 % Feuchtigkeit basierend) und eine Lichttransmission (Klarheit) von 94,2 % auf.

### Beispiel VI

### Carboxymethylierung

350g einer +M20-Fraktion mit einem Feuchtigkeitsgehalt von 7,5 % wurden mit 100ml kaltem Wasser befeuchtet. Nach 15 Minuten wurde der grösste Teil der Monochloracetat-Na-Lösung, bestehend aus 79 g Monochloracetat-Na und 184 g Wasser während 24 Minuten unter Rühren langsam zugegeben Die übrigen 100 ml dieser Lösung wurden während 5 Minuten schnell zugegeben. Die Zugabe der Reagenzien erfolgte bei Raumtemperatur.

Nach weiteren 16 Minuten Inkubation unter Rühren wurde die Temperatur auf 50° C erhöht. Nun wurden 27 g NaOH-Plätzchen zugegeben. Aufgrund der exothermen Reaktion der verwendeten NaOH-Plätzchen stieg die Temperatur schnell auf 65-66° C an und wurde 36 Minuten beibehalten.

Das Reaktionsprodukt wurde aus dem Reaktor entfernt und zweimal mit Wasser in einem Verhältnis von 1:6 gewaschen. Aufgrund der sehr schnellen Wasseraufnahme der gereinigten, nicht mit Borax behandelten Carboxymethyl-Splits, wurde deren Verweildauer in Wasser jeweils auf 2 Minuten beschränkt. Das Gewicht der stark gequollenen Splits betrug 2640 g. Daher war ein Entwässerungsschritt mit Isopropanol (1500g wurden verwendet), notwendig.

3261 g des Filtrats wurden zurückgewonnen. Das Gewicht der mit Alkohol befeuchteten, carboxymethylierten Splits betrug 879 g mit einer flüchtigen Menge von 66,8 %.

Da keine Vorsichtsmassnahmen während der Reaktion durch eine inerte Stickstoffhaube getroffen wurde, waren die Splits während der gesamten Reaktionszeit mit Luft in Kontakt, was eine beträchtliche Depolymerisation verursachte.

Die Viskosität einer 1 %igen-Lösung der carboxylierten Splits betrug 1720 mPa.s bei einer Klarheit von 92,7 %. Diese Werte wurden bei einer Temperatur von 25° C erhalten.

### Beispiel VII

400 g der bevorzugten Guarsplitqualität mit einem Galaktomannangehalt von mindestens 84 % wurden in einem vorgeheizten Sigma-Mischer zuerst mit 4 ml 35 % H₂O₂, verdünnt, mit 21 ml entmineralisiertem Wasser während 9 Minuten behandelt, wonach 40 g NaoH, gelöst in 61 ml entmineralisiertem Wasser heiss zugesetzt wurden.

Innerhalb von 3 Minuten erreichte das Reaktionsgemisch die erwünschte Temperatur von 68° C. Die Reaktionstemperatur von 68° - 72° C wurde 20 Minuten aufrecht gehalten. Anschliessend wurde dem Reaktionsgemisch das meiste Wasser durch Ventilation und indirekter Erhitzung während 23 Minuten entzogen.

Das Reaktionsgemisch wurde über M20 gesiebt, wobei 30g -M20 und 437 g +M20 (H₂O-Gehalt 10,1 %) erhalten wurden. Die +M20-Fraktion wurde zweimal mit Wasser gewaschen, wobei jeweils 6 Teile Leitungswasser auf 1 Teil der ungewaschenen +M20-Fraktion verwendet wurden. Die jeweiligen Waschzeiten waren 4 und 5 Minuten.

Die gewaschenen, gequollenen Splits wurden durch einfaches Sieben zurückgewonnen. 892 g gequollene Splits wurden erhalten, die einen Wassergehalt von 67,8 % aufwiesen. Diese Splits wurden wie üblich in Lösung gebracht und diese Lösung erzeugte eine Viskosität von 2300 mPa.s und eine Lichttransmission von 86,4 % (gemessen nach Beschreibung in Beispiel I).

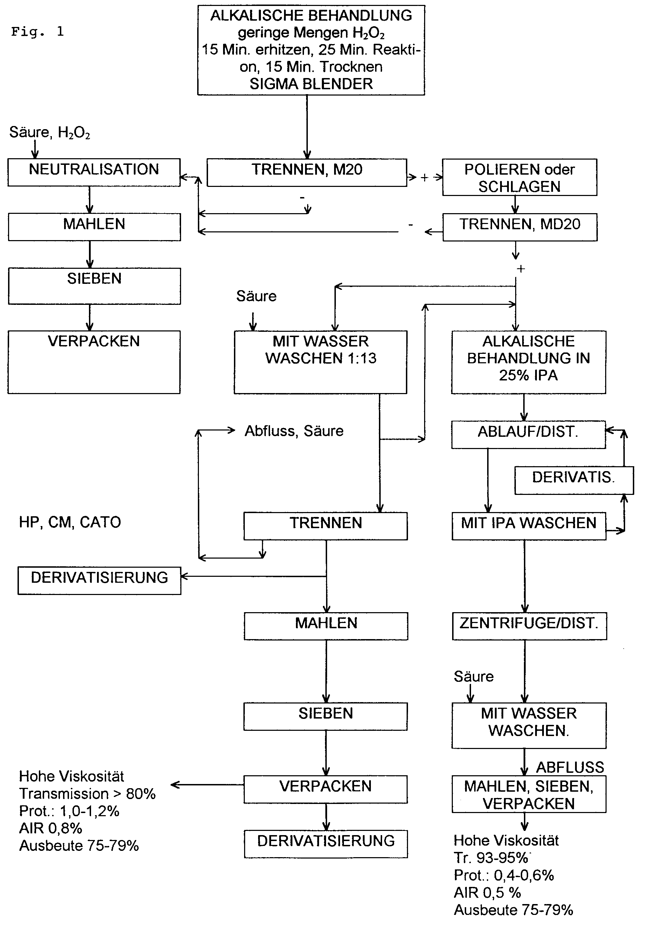

Fig. 1: Das Flussdiagramm zeigt die erfindungsgemässe Behandlung der Splits mit Lauge und Wasserstoffperoxid und die sich an die alkalische Behandlung anschliessenden Weiterbearbeitungsmöglichkeiten.

## Patentansprüche

1. Verfahren zur Herstellung von reinen Guarkernmehl, dadurch gekennzeichnet, dass es die folgenden Schritte umfasst:
(a) Behandeln der Guar-Splits mit einer alkalischen Lösung in Anwesenheit von Wasserstoffperoxid bei 65 bis 70 °C;
(b) teilweise Neutralisation der alkalischen Splits mit einer organischen oder anorganischen Säure;
c) mechanisches Entfernen der peripheren Zellschichten der Gu ar-Splits; und
(d) Behandeln der Splits mit einer wässrigen Alkohollösung,
(e) Vermahlen der Splits zu Mehl.

2. Verfahren gemäss Anspruch 1, dadurch gekennzeichnet, dass die im Schritt (a) verwendete alkalische Lösung Natronlauge ist, vorzugsweise in einer Konzentration von 25 bis 50 Gewichtsprozent, am meisten bevorzugt in einer Konzentration von 33 Gewichtsprozent und in einer Menge von 6 bis 10 % basierend auf dem Ausgangsmaterial.

3. Verfahren gemäss Anspruch 1 oder 2, dadurch gekennzeichnet, dass die im Schritt (a) verwendete Menge Wasserstoffperoxid 0,175 bis 0,35 Gewichtsprozent , am meisten bevorzugt 0,175 Gewichtsprozent umfasst, wobei eine Wasserstoffperoxidlösung von 35 Gewichtsprozent verwendet wird.

4. Verfahren gemäss einem der vorstehenden Ansprüche, dadurch gekennzeichnet, dass die Säure in Schritt (b) Schwefelsäure, Phosphorsäure oder eine andere geeignete organische Säure, vorzugsweise Eisessig, ist.

5. Verfahren gemäss Anspruch 4, dadurch gekennzeichnet, dass die in Stufe (b) verwendete Schwefelsäure in einer Konzentration von 60 bis 80 Gewichtsprozent, vorzugsweise von 70 Gewichtsprozent verwendet wird.

6. Verfahren gemäss Anspruch 4, dadurch gekennzeichnet, dass die in Stufe (b) verwendete Phosphorsäure in einer Konzentration von 60 bis 85 Gewichtsprozent, vorzugsweise von 75 Gewichtsprozent verwendet wird.

7. Verfahren gemäss einem der vorstehenden Ansprüche, dadurch gekennzeichnet, dass die in Schritt (d) verwendete Alkohollösung eine wässrige Lösung von Ethanol, Methanol oder vorzugsweise Isopropanol ist.

8. Verfahren gemäss Anspruch 7, dadurch gekennzeichnet, dass die wässrige Alkohollösung vorzugsweise eine 20 bis 40 gewichtsprozentige, am meisten bevorzugt eine 25 gewichtsprozentige Isopropanollösung ist.

9. Verfahren gemäss Anspruch 7 oder 8, dadurch gekennzeichnet, dass die Splits nach Entfernen der peripheren Zellschichten in Schritt (c) und vor der Behandlung mit einer wässrigen Isopropanollösung in Anwesenheit einer Lauge, vorzugsweise Natronlauge, bei 60 bis 70 °C in Schritt (d) zweimal mit Wasser gewaschen werden.

10. Reines Guarkernmehl gemäss einem der vorstehenden Ansprüche, dadurch gekennzeichnet, dass genanntes Guarkernmehl einer Derivatisierung durch Carboxymethylierung, Hydroxypropylierung oder Kationisierung oder Kombinationen davon unterzogen wird.

11. Guarkernmehl, das als 1%-ige wässrige Lösung eine Viskosität bis zu 9000 mPa.s aufweist, das einen Gehalt an Proteinen und durch Säure nicht hydrolisierbare Stoffe von zusammen von 0,8 bis 1,2 % besitzt und als 0,5%-ige wässrige Lösung eine bei einer Wellenlänge von 500 nm gemessene Transparenz von mindestens 80 % zeigt, hergestellt nach dem Verfahren gemäss einem der vorstehenden Ansprüche.

12. Guarkernmehl gemäss Anspruch 10, dadurch gekennzeichnet, dass die bei einer Wellenlänge von 500 nm gemessene Transparenz einer 0,5 %-igen Lösung dieser Derivate bis zu 98 % ist.

13. Guarkernmehl gemäss einem der Ansprüche 10, 11 und 12 zur Verwendung als Verdickungsmittel für Lebensmittel, für Kosmetika, für pharmazeutische Produke, für Farben und Lacke, für Textilfarben, für die Erdölgewinnung sowie für explosive Stoffe.
